# NEW EUROPEAN PATENT SPECIFICATION

(11) **EP 2 073 647 B2**
(45) Date of publication and mention of the opposition decision: **13.08.2014**
(45) Mention of the grant of the patent: 05.05.2010
(21) Application number: 07834629.3
(22) Date of filing: 10.10.2007
(51) Int. Cl.: A23L 1/29, A23L 1/30, A23L 1/09

(54) **KETOGENIC DIET**
KETOGENE DIÄT
DIÈTE CÉTOGÈNE

(30) Priority: 17.10.2006 US 581749
(43) Date of publication of application: 01.07.2009
(73) Proprietor: N.V. NUTRICIA, 2712 HM Zoetermeer (NL)
(72) Inventor: BUTLER, Raelene, Liverpool L17 0HG (GB); HAGEMAN, Robert Johan Joseph, 6705 CT Wageningen (NL)
(74) Representative: Nederlandsch Octrooibureau
(86) International application number: PCT/NL2007/050491
(87) International publication number: WO 2008/048094

(56) References cited:
- EP-B1- 1 121 865
- WO-A-01/95739
- WO-A-94/02166
- WO-A-2006/118665
- WO-A1-2008/048094
- WO-A2-2005/039597
- US-A- 4 544 559
- US-B2- 6 913 778
- JOURNAL OF THE AMERICAN MEDICAL ASSOCIATION, vol. 290, no. 7, 20 August 2003 (2003-08-20), pages 912-920, XP002468152 USA
- ZUPEC-KANIA ET AL.: 'Clinical Use of the Ketogenic Diet', 2004, HUMANA PRESS, TOTOWA pages 63 - 79
- HOSAIN ET AL.: 'Ketogenic Diet in Pediatric Epilepsy Patients with Gastronomy Feeding' PEDIATRIC NEUROLOGY vol. 32, no. 2, 2005, pages 81 - 83
- Abbott Nutrition, RCF formula
- Abbott Nutrition, Polycose
- MIKE ET AL.: 'Practical Guide and Dietary management of Children with Seizures Using the Ketogenic Diet' AMERICAN JOURNAL OF CLINICAL NUTRITION vol. 17, 1965, pages 399 - 409
- 'The ketogenic diet: A practical guide for caregivers' JOURNAL OF THE AMERICAN DIETETIC ASSOCIATION vol. 98, no. 3, March 1998, pages 316 - 321
- KWITEROVICH ET AL.: 'Effect of a High-Fat Ketogenic Diet on Plasma Levels of Lipids, Lipoproteins, and Apolipoproteins in Children' AMERICAN MEDICAL ASSOCIATION vol. 290, no. 7, 20 August 2003, pages 912 - 920
- CASEY ET AL.: 'The implementation and Maintenance of the Ketogenic Diet in Children' JOURNAL OF NEUROSCIENCE NURSING vol. 31, no. 5, October 1999, pages 294 - 302
- FREEMAN ET AL.: 'The Ketogenic Diet - a treatment for children and others with epilepsy', vol. 4, 2006, DEMOS MEDICAL PUBLISHING, NEW YORK pages 173 - 181
- Website of Demos Medical Publishing with bibliographical data on D10
- SHS North America "KetoCal - Dietitian's Guide", product information, 2006 ¬"06/05 ZKETDG-E|
- Delivery note nr. 66343 dated 02-06-2004, issued by Dr. Sulewack
- Product specification of Dr. Sulewack pertaining to article nr. 80436, dated 17-08-2009
- KRIST ET AL.: 'Lexikon der pflanzlichen Fette und Öle', 2008, SPRINGER-VERLAG, WIEN page 333
- TAHA ET AL.: 'Despite transient ketosis, the classic high-fat ketogenic diet induces marked changes in fatty acid metabolism in rats' METABOLISM CLINICAL AND EXPERIMENTAL no. 54, 2005, pages 1127 - 1132
- SCHLANGER ET AL.: 'Diet Enriched with Omega-3 Fatty Acids Alleviates Convulsion Symptoms in Epilepsy Patients' EPILEPSIA vol. 43, no. 1, 2002, pages 103 - 104
- USPTO office action relating to application no. 11/581,749
- SOUCI ET AL.: 'Food Composition and Nutrition Tables 1989/90', 1989, WVG, STUTTGART pages 876 - 877
- DEBInet, "Pecannuss geröstet", Internet website printed on 24-01-2012
- DEBInet, "Paranuss", Internet website printed on 24-01-2012
- Declaration by Mr Tobias Nolte, referring to D13 and D14
- Souci, Fachmann, Kraut, p. 1024-1025, nutritional data for Brazil nut
- Souci, Fachmann, Kraut, p. 1026-1027, nutritional data for Pecan nut.
- SOUCI ET AL.: 'Data on the composition of cream and soybean, oil', 1989, WISSENSCHAFTLICHE VERLAGSGESELLSCHAFT MBH, STUTTGART
- Article from Wikipedia on fatty acids, referring on particular to essential fatty acids
- Third party observations filed on 19-03-2012
- 'Report of a Joint FAO/WHO/UNU Expert Consultation' HUMAN ENERGY REQUIREMENTS 17 October 2001, pages 1 - 96
- MAGRATH ET AL.: 'Dietary practices and use of the ketogenic diet in the UK' JOURNAL OF THE BRITISH EPILEPSY ASSOCIATION vol. 9, no. 2, March 2000, pages 128 - 130
- KELLER ET AL.: 'Evaluation und Behandlung der Adipositas in der Praxis' THERAPEUTISCHE UMSCHAU vol. 46, no. 5, 19 March 2012, pages 297 - 308
- Vining, Clinical efficacy of the ketogenic diet, Epilepsy Research, December 1999, 373(3), p. 181-90, Medline abstract
- Campbell et al., Nutritional Management of Glucose Transport Protein Deficiency Type 1 (Glut I deficiency) using Ketocal 3:1 (SHS), Sept 2008
- KetoCal 3:1 ex Nutricia, nutritional data and ingredient list in Dutch language
- KetoCal 3:1 ex Nutricia, nutritional data and ingredient list in English language

## Description

### Field of the invention

The invention is related to a single palatable product that can be used as ketogenic diet, that is suitable for enteral use by epileptic patients from birth to 12 months of age, and that comprises low amounts of trans fatty acids, a high energy density and a relatively high amount of protein.

### Background

The human body derives the energy that is needed predominantly from eating and metabolizing proteins, lipids and digestible carbohydrates. In the typical Western diet, digestible carbohydrates provide most of the energy, in most cases more than 50 energy percent, using 4 kcal or 16.8 kJ per gram as the conversion factor for digestible carbohydrates and proteins and 9 kcal or 37.8 kJ per gram for lipids. Metabolism of digestible carbohydrates predominantly releases glucose, which is a preferred energy source for the human body. Under special conditions most human cells can also use other organic compounds as energy source, such as amino acids, fatty acids and ketones. It is generally considered that increasing the amount of carbohydrates relative to that of lipids decreases the ketogenic properties of the product. Therefore classical ketogenic products for paediatric epileptics provide 4 times more lipids than the sum of proteins and digestible carbohydrates calculated on a weight base.

A ketogenic diet is a diet, which provides, after consumption, digestion and metabolism, ketones as a major energy source. These ketonic compounds include in particular aceto acetate, D-3 hydroxy butyrate and acetone. Keto-acids like oxaloacetate are not calculated as ketobodies. In particular a ketogenic diet comprises more than 60 g lipids per 100 g dry mass of the dietetic products, so more than 77 energy percent.

Ketogenic diets have been used for treatment of epileptic convulsions, and in treatments of obesity and weight management. Such diets may be composed of a variety of different meals which each fit within the diet, or may consist of one single product, which can be used for complete nourishment of a human being, when used as the sole nutrition. A product called Ketocal belongs to the latter category and is used for combating epilepsy, in particular intractable epilepsy in young infants. The product provides per 100 g dry matter 3011 kilojoules and comprises per 100 g dm 15.25 g protein, 73 g lipids and 3 g digestible carbohydrates. The amount of saturated fatty acids is about 22 wt% of the triglycerides. However, the extremely low amount of carbohydrates and the nature of the lipid made the product not optimal for human consumption. It is therefore an object of the current invention to provide a highly effective product that is better palatable, while providing a better lipid fraction in terms of ketogenic properties, tolerance, safety and a lower amount of trans fatty acids.

EP 0843972 discloses a complete product for enteral feeding persons suffering from the metabolic syndrome or hypertriglyceridaemia, which comprises 33-63 energy percent fats, and the proteins 5-30 en% of the composition and in which the fatty acids comprise 55-90 wt% medium chain fatty acids, 5-25 wt% polyunsaturated fatty acids and 0-30 wt% other fatty acids. The levels of palmitic acid are very low, in the order of 0.5-1 % on fatty acid basis.

The problem underlying the invention is thus to provide a nutritional formula, which is suitable for use as complete nutrition when administered as dry, semi-solid or as a reconstituted liquid product, and which is especially intended for meeting the needs of epileptic infants, providing high levels of ketogenic components, while avoiding the drawbacks of the prior art products.

### Description of the invention

This problem has been solved according to the invention by increasing the amount of digestible carbohydrates and selecting particular lipids as ingredients. These lipids not only provide the right type of energy when catabolized, but also provide relatively high amounts of palmitic acids which can be used for biosynthetic purposes, in particular in pediatric epileptics, who typically suffer from growth retardation.

The product according to the invention is a product that is suitable for complete nourishment of human beings, in particular infants and more in particular pediatric epileptic patients. The product therefore comprises a protein fraction, a lipid fraction, a fraction of digestible carbohydrates, a vitamin fraction and a mineral/trace element fraction and optionally other components.

The protein fraction comprises preferably peptides larger than 8 amino acids, which makes the product unsuitable for parenteral administration, due to potential allergic reactions. It is preferred to include proteins in the formula, which have strong emulgating properties, such as certain caseins, which are known in the art. When partially hydrolyzed proteins are used, it is preferred to include at least 20% hydrolyzed whey as indicated below, in order to meet nutritional requirements. However, products usable for reconstitution in water or liquid formula should preferably contain lyso-lecithin, tartaric esters or combinations thereof as stabilization system in order to obtain a product that is suitable for drinking

The product is a powder, which can be reconstituted in water to be used as a single complete food. The powder can consist of primary particles, but also of agglomerated primary particles or mixtures of particles of various size. Such powders can be manufactured using methods known in the art, such as spray drying, especially when aids are used to improve flowing characteristics.

Useful liquids for feeding pediatric epileptic patients have an energy density of .9- 3.0 kcal/ml (3.8- 12.6 kJ/ml) preferably 1.1-2.0 kcal/ml (4.6- 8.4 kJ) and more preferably 1.2-1.7 (5.0-7.2 kJ/ml). Especially 1.3-1.6 kcal/ml (5.4- 6.7 kJ/ml) appears useful when nourishing completely with the formula. When the energy density of the liquid formula is 8.4-12.6 kJ/ml, the product is also useful for intermediate fortification of the patient.

When applying the liquid, or solid product as intermediate fortifier between meals, the product beneficially comprises per 100 kiloJoule as provided by the product more than 4.0, preferably more than 8, more preferably 20-1000 mg of an added methyl donor, selected from the group of serine, choline, betaine, dimethylglycine, sarcosine, MSM and SAM.

The dry product is at least partly soluble in water, so as to allow ready make-up of a liquid food, if desired. Preferably at least 50 wt%, more preferably at least 75 wt% of the dry mass is soluble when dissolved as 10% (w/v) in water at 20 °Celsius.

The product is relatively energy dense for products for infants. In particular it provides 600-735 kcal or 2520-3080 and preferably 2800-3040 kJ per 100 grams dry matter. The weight ratio of lipids to protein and digestible carbohydrates is in the range of 2.7-3.4 and preferably 2.7 to about 3 to one.

The amount of digestible carbohydrates is 3.2-9, preferably 4-8.6, more preferably 5-8.2 g per 100 g dry mass. The amount of protein is 13-18, preferably 13.8-17, more preferably 14.2-16.2 g per 100 g dry mass. The amount of lipids is 60-80, preferably 63-75, more preferably 65-72 g per 100 g dry mass.

The protein comprises all essential amino acids and in particular relatively high amounts of lysine, leucine and the sulphur amino acids methionine or cysteine, in order to avoid or even counteract growth retardation when providing a ketogenic diet.

Per 100 g protein the amount of leucine is typically more than 9.4 g and preferably more than 10.2 g. Lysine should preferably be included in an amount of more than 7.7, more preferably more than 7.9 and most preferably more than 9.1 wt.% of the protein. Of cysteine suitably more than 1.8, preferably more than 2.2 and even more preferably more 2.4 g is included per 100 g protein in order to facilitate lipid use. The same applies to methionine, i.e. preferably more than 1.8 g, preferably more than 2.2 and even more preferably more than 2.4 g is included etc. per 100 g protein.

The inclusions of alpha-lactalbumin or ingredients which comprise high amounts of this protein, are particularly suitable. The presence of more than 20 wt% alpha-lactalbumin in the protein fraction of the product results in easy compliance with the requirements for leucine, lysine, methionine and cysteine, excellent palatability and digestion properties. Preferably more than 20%, more preferably 40-80 wt% of the protein fraction consists of alpha- lactalbumin.

The lipid fraction can comprise triglycerides, diacyl-glycerides, monoacyl-glycerides, phospholipids, lyso-phospholipids, cholesterol and glycolipids. Though excellent products are obtained in terms of efficacy, safety and palatability, by including more than 97% triglycerides as lipid source, the digestibility can be improved by replacing part of the triglycerides by phospholipids, lyso-phospholipids, diacyl-glycerides and/or monoacyl-glycerides. This avoids constipation, and increases the bioavailability of divalent cations, in particular calcium, magnesium and zinc. Replacement of at least 10% and preferably more than 14%, up to e.g. 25% or preferably up to 20% (weight basis) of the triglycerides with one or more of phospholipids, lyso-phospholipids, diacylglycerides and monoglycerides appears to be beneficial in this respect. The amount of phospholipids should be less than 14%, preferably less than 8% of the lipid fraction, in order to prevent too high phosphorous levels, which may impart digestibility of divalent cations.

The lipid fraction must comprise linoleic acid and alpha-linolenic acid. These together provide more than 15 wt% in particular more than 17 wt.% of the lipids (on fatty acid basis). Linoleic acid provides more than 14, preferably 15-25, in particular 16-21 energy % of the composition (using also the factor 37.8 kJ/g fatty acid). Alpha-linolenic acid must provide at least 1.2 energy% of the composition.

The amount of polyunsaturated fatty acids (i.e. having two or more unsaturated bonds), to which the trans fatty acids have been excluded, is 16-40, preferably 20-30 g per 100 g lipids. It is preferred that the lipid fraction also comprises ω-3 polyunsaturated fatty acids other than alpha linolenic acid. In particular, the polyunsaturated fatty acids comprise more than 0.5, preferably 1.0-10 wt.% of docosahexaenoic acid. It is even more preferred to include in the polyunsaturated fraction also arachidonic acid in an amount of more than 1.0 wt.%, up to 6 wt.% of the polyunsaturated fatty acid fraction. The amount of trans fatty acids is below 20, preferably 0-10, more preferably 0.2-4 g per 100 g lipids.

The amount of saturated fatty acids is relatively high. In particular it must be between 23 and 50, preferably 25-45, more preferably 33-44 g per 100 g lipids, on fatty acid basis. The saturated fatty acids have 8 to 24 carbon atoms. It is preferred that a major part of the saturated fatty acids is palmitic acid (C16:0). Palmitic acid thus provides e.g. 15-50, preferably 18-45, more preferably 23-44 g per 100 g lipids. A particular embodiment comprises 30-37 g palmitic acid per 100 g lipids. Therefore, palm oil is a preferred source for at least 50%, preferably between 70 and 90 of the lipid fraction. The remainder of the lipid fraction can be selected from e.g. safflower, sesame, soybean or sunflower oil or mixtures thereof, preferably soybean oil (preferably between 2 and 30), medium-chain triglycerides (with fatty acids having 8-14 carbon atoms; between 0 and 14), marine oils (preferably between 0 and 14 wt.%, more preferably between 2 and 12 wt.%), and phospholipids, mono- and di-glycerides.

The amount of mono-unsaturated fatty acids is suitably between 25 and 48, preferably 28-43, more preferably 30-40 g per 100 g lipids (fatty acid basis).

A blend of oils containing one or more ingredients, preferably containing at least 2, 3, 4, 5, most preferably all of those ingredients as given in Table 1 has been found to be very suitable for use in the manufacture of a ketogenic diet.

**Table 1: Blend of ingredients for use in a ketogenic diet**

| | Effective | Preferred | More pref. | Most pref. |
|---|---|---|---|---|
| palm oil(s) | 70-90 | 70-90 RPO | 75-85 PO | 75-85 RPO |
| vegetable oil(s) | 2-30 | 2-30 RSBO | 10-20 VO | 10-20 SBO |
| MCT oil | 0-14 | 0-14 | 0-5 | 1-4 |
| LCPoil (s) | 0-14 | 0-14 | 1-12 of 2 oils | 1-6 AO+ FO |
| PL's | 0-14 | 0-14 | 1-8 lecithins | 3-7 SBL |
| MG or DG | 0-14 | 0-14 | 1-20 DG | 1-20 DG |

| | | | | |
|---|---|---|---|---|
| Numbers expressed as weight % of the lipid fraction The various abbreviations will be explained in the text below | | | | |

Palm oils (PO) are defined to be food grade oils, soluble in diethyl ether, and originating from the palm tree and include crude oils from palm fruits/nuts or palm kernel, which comprise > 10% of non-triglycerides compounds (NTG) like carotenoids, terpenes like squalane or squalene, tocopherols and steroids, and refined oils (RPO) from the nuts or kernel or mixtures thereof. NTG is therefore not water or dirt, ash or cell wall material.

Vegetable oils (VO) are defined to be the crude or defined food grade oils originating from soybean, safflower, rapeseed, sunflower or linseed. SBO originates from soybean and may comprises various amounts of non-triglyceride material such as phospholipids. Refined SBO (RSBO) comprises less than 10% non-triglyceride compounds (NTG).

MCT oil are food grade oils known in the art, and defined to comprise more than 90wt% of the fatty acids being saturated fatty acids having 8, 10 or 12 carbon atoms.

Phospholipids (PL's) can originate from sources known in the art such as egg, soybean or rapeseed and can provide mixtures of phosphatidyl cholines and phosphatidyl ethanolamines and optionally phosphatidyl inositols or phosphatidyl serines.

LCP oil is defined to be any food grade oil that comprises more than 15% of fatty acids having 20 or more carbon atoms. In particular these oils comprise more than 15% docosahexaenoic acid or arachidonic acid. Suitable sources of such oils are marine oils, such as fish oil (FO), algal oils or yeasts, fungi or organisms that are genetically modified to biosynthesize these fatty acids, such as bacteriae, yeasts, plants, or eggs from specifically fed or modified poultry.

The use of these lipids appeared to provide a suitable fatty acid profile, low amounts of trans fatty acids and allowed manufacture of products that were well tolerated by epileptic infants or adults.

The digestible carbohydrate fraction can comprise food grade ingredients such as glucose syrup, maltodextrins, lactose, sucrose, galactose, ribose, etc. Though excellent products can be obtained in terms of efficacy when several of the other technical features as disclosed in this description are applied, best results in terms of avoidance of side effects and efficacy are obtained if the digestible carbohydrate fraction takes a specific form. It appears beneficial if at least 20%, preferably 30-90% of the digestible carbohydrate fraction is formed by a source of galactose or ribose. Lactose is considered as suitable ingredient for this purpose. In particular oxidative stress will decrease, when such ketogenic formula is consumed, which comprises such non-glucose digestible carbohydrates.

The digestibility is determined by applying the Englyst 1999 method.

The proteins, lipids, and carbohydrates, preferably originate from at least two different sources, for example, the proteins at least partly from animal, especially milk, source, but optionally also partly from plant source, the lipids at least partly from vegetal source, and the carbohydrates at least partly from milk source, or from a combination of milk (lactose) and plant (glucose, maltodextrins etc.).

The amount of micro ingredients, which must be included in the product follow recommendations for infants. However, increasing the amounts of several specific ingredients above recommendations improves efficacy and prevents side effects in pediatric epileptics

For example the amount of vitamin B6 must be above 0.8 mg, preferably 0.9-90 mg, most preferably 1.0-60 mg per 100 g dry product, in order to improve efficacy, and decrease negative side effects. This is especially applicable when the product is used in pediatric epileptic patients that do not respond to anti-convulsants. Suitable sources include pyridoxine, pyridoxamine and pyridoxal including phosphates or salts of these components. However, the use of pyridoxal is preferred.

Biotin is another preferred ingredient of the composition of the invention. Infants require 6 - 200, preferably 8 - 100, more preferably 9 - 50 µg per daily dose. Such daily dose is typically provided by 100 g powder, which is reconstituted in water to result in 500 ml food (about 20% solution) for a 2 month old baby. At 6 months of age this daily dose will be increased to 200 g powder. In situations wherein the disorder has a chronic nature and the enteral product is consumed on a daily basis, like many persons suffering from the metabolic syndrome or diabetes type II will do in order to prevent or treat increased plasma ketone levels, the amounts of biotin are preferably 50 - 1000, more preferably 70 - 500, even more preferably 80 - 300 µg per day for children older than 11 years and adults and 10 - 200, more preferably 15 - 150, even more preferably 18 - 100 µg per day. In acute situations like acidosis, the amounts are preferably higher. For example children above 11 years of age and adults will require 300 - 20000, preferably 360 - 2000, more preferably 420 - 1000 µg per daily dose. Infants of younger age need 40 - 500, preferably 50 - 250, more preferably 60 - 150 µg. Premature infants should be administered 9 - 200, preferably 12 - 100 and more preferably 15 - 50 µg per daily dose.

The composition thus preferably contains biotin in an amount of 6 - 1000 µg, preferably 8 - 400 µg per 100 g dry matter. For general formula's, the preferred amount is 9-50, in particular 12- 50 µg per 100 g. For special purposes, such as acidosis, the preferred amount is 40-400, especially 50-250 µg per 100 g dry matter.

Biotin in the amounts according to the invention is found to decrease the levels of ketone bodies in blood plasma, the levels of AGE products and Maillard products in tissue, and the degree of acidosis in a shorter time, e.g. lactic acidosis but also ketonic acidosis, and to normalize the lipid profile, in particular cholesterol plasma levels.

Vitamin B12 can be provided using suitable sources, such as synthetic Cyanocobalamin, methyl cobalamin, adenosyl cobalamin and hydroxyl cobalamin, for instance obtained from isolates of organs, in particular the liver, e.g. a aqueous concentrate of lysed hepatocytes of agricultural animals, e.g. pig, cow, chicken, with a concentration higher than 75 µg cobalamines per 100 ml extract. The composition preferably contains vitamin B12 in an amount of 0.25 - 50 µg per 100 g of the dry composition, more preferably 0.4 - 10 µg and most preferably 0.8 - 5 µg per 100 g of dry matter.

The amount of pantothenic acid is preferably above 3, more preferably 4-20, most preferably 4.5-14 mg per 100 g dry matter.

Per 100 g dry product more than 25 mg L-carnitine and preferably 28-200 mg is included in order to improve energy generation from the product. Very suitable ingredients are the alkylated carnitines, especially N-acetyl carnitine or N-propyl or N-isopropyl carnitine.

The amount of calcium in the formula is relatively high, i.e. more than 580 and preferably 600- 900, more preferably 620-750 mg per 100 g dry mass. In order to allow a high bioavailability of the calcium, the ratio calcium to phosphorous is more than 1.1 : 1 and more preferably 1.2-2 : 1. Calcium salts are preferably soluble salts, such as the salts with organic anions.

The amount of taurine is advantageously more than 35, preferably 40-400, more preferably 45-200 mg per 100 g dry product. Suitable taurine sources are known in the art and include taurates.

The product beneficially includes a source of nucleotides, nucleosides or nucleobases. These sources include synthetic compounds such as uridine, cytidine, adenine including their salts, as well as their phosphates, such as uridine monophosphate, uridine diphosphate as well as extracts from yeast, algal -, bacterial- or other cultures or tissue, which provide such components. Very suitable sources include UMP, CMP and cyticoline. Nucleotides or equivalent compounds are preferably included in an amount of 2-600, preferably 6-100, most preferably 10-35 mg per 100 g dry mass. Most preferably uridine is included in an amount of 2-50, preferably 4-30, most preferably 7-12 mg per 100 g dry powder.

The product also beneficially comprises oligosaccharides. In particular in an amount of 10 mg- 2 g , preferably 40 mg- 1.8 g per 100 g dry product. Suitable oligosaccharides include those having a chain length of 3-20 monosaccharide units. Such oligosaccharides preferably comprise at least two but preferably at least three different monosaccharides, of the group galactose, fructose, fucose, mannose, arabinose, glucuronic acid, galacturonic acid, sialic acid, N-acetyl glucosamine. Such mixtures can be obtained by inclusion of pure oligosaccharides or inclusion of at least two mixed oligosaccharides, for example β-galacto-oligosaccharides and fructo-oligosaccharides. Inclusion of such oligosaccharides decreases the immunological problems in patients that use ketogenic diets as single nourishment.

The product is effective in improved seizure control, in particular in terms of a lower frequency of seizures in short and long term, and in terms of a decreased severity of seizures. Especially infants that have no inborn errors of metabolism, except infants suffering from defects in their glucose transporters, and infants that were irresponsive to anti-convulsant therapy benefit, from the formula. Undesired side effects in terms of growth retardation, metabolic acidosis, decreased immune function, kidney problems and constipation are relatively low.

The product appeared also useful when applied in a weight management regimen according to the views of Dr. Atkins for adults having a BMI above 25 and other obese persons and especially in obese persons suffering from the metabolic syndrome or from insulin resistance. This is in particular true when the product is used as single nutrition and preferably when the compositions according the invention are consumed as several ready to use products of which at least two possess a differing physical shape in terms of being liquid, semi solid or solid.

The product, suitably after reconstitution to a liquid product, can be administered in an amount of between 50 and 200, preferably between 75 and 150 g per day, calculated as dry mass, for infants younger than 12 months, following general energy consumption recommendations, like has been described in the guidelines of the health authorities (e.g. Gezondheidsraad 2000). For older children ,the preferred daily amount calculated on dry mass is between 100 and 360, especially between 150 and 300 g. For adults these amounts are 100-500, most preferably 150-340 g for providing most energy as ketone bodies, and at the same time a sufficient amount of essential amino acids, carbohydrate skeletons, and other nutrients and being well tolerated and safe.

### Example 1: anti-epileptic formula for paediatrics

| ***Nutrition Information*** | | | | ***Per 100g Powder*** | ***Per 100 kcal* *** | ***Per 100ml***** |
|---|---|---|---|---|---|---|
| Energy | | | kJ | 2918 | 413 | 292 |
| | | | kcal | 707 | 100 | 70.7 |
| Protein | | | g | 15.2 | 2.1 | 1.5 |
| Carbohydrate | | | g | 7.6 | 1.1 | 0.76 |
| | as sugars | | g | 0.68 | 0.1 | 0.07 |
| Fat | | | g | 68.4 | 9.7 | 6.8 |
| | of which saturates | | g | 25.4 | | |
| | | monounsaturates | g | 23.6 | | |
| | | polyunsaturates | g | 16.4 | | |
| | % LCT | | | 100 | | |
| | Ratio ω-6 : ω-3 fatty acids | | | 13.6 | | |
| | % energy from linoleic acid | | | 19.2 | | |
| | % energy from α linolenic acid | | | 1.4 | | |
| Fat: protein + carbohydrate | | | (g/g) | 3 : 1 | | |
| Fiber | | | | nil added | | |

| | | | | | | |
|---|---|---|---|---|---|---|
| * approximately 14.1g powder ** 109 made up to 100mls | | | | | | |

| ***Typical Amino Acid Profile*** | ***g*/*100g Powder*** |
|---|---|
| L-Alanine | 0.5 |
| L-Arginine | 0.53 |
| L-Aspartic Acid | 1.1 |
| L-Cystine | 0.42 |
| L-Glutamic Acid | 3.3 |
| Glycine | 0.29 |
| L-Histidine | 0.46 |
| L-Isoleucine | 0.75 |
| L-Leucine | 1.5 |
| L-Lysine | 1.2 |
| L-Methionine | 0.43 |
| L-Phenylalanine | 0.76 |
| L-Proline | 1.6 |
| L-Serine | 0.81 |
| L-Threonine | 0.73 |
| L-Tryptophan | 0.41 |
| L-Tyrosine | 0.81 |
| L-Valine | 0.99 |
| L-Carnitine | 0.03 |
| Taurine | 0.05 |

| ***Carbohydrate Profile*** | ***g* / *100g Carbohydrate*** | ***g* / *100g Powder*** |
|---|---|---|
| Dextrose | 1.9 | 0.14 |
| Lactose | 0.4 | 0.03 |
| Maltose | 6.7 | 0.51 |
| Maltotriose | 9.5 | 0.72 |
| Higher Saccharides | 81.5 | 6.2 |

| ***Typical Fatty Acid Profile*** | ***g* / *100g Fatty Acids*** |
|---|---|
| C_{16:0} | 34.6 |
| C_{16:1} | 0.2 |
| C_{18:0} | 4.2 |
| C_{18:1} | 35.8 |
| C_{18:2} | 23.1 |
| C_{18:3} | 1.7 |
| C_{20:1} | 0.1 |
| C_{20:2} | 0.3 |

| ***Vitamins*** | | ***Per 100g Powder*** | ***Per 100 kcal* *** | ***Per 100ml***** |
|---|---|---|---|---|
| Vitamin A | µg RE | 781 | 110 | 78.1 |
| | IU | 2601 | 368 | 260 |
| Vitamin D | µg | 14.1 | 2 | 1.4 |
| | IU | 564 | 79.8 | 56.4 |
| Vitamin E | mg α T.E. | 8.9 | 1.3 | 0.89 |
| | IU | 13.3 | 1.9 | 1.3 |
| Vitamin C | mg | 134 | 19 | 13.4 |
| Vitamin K | µg | 29.8 | 4.2 | 3 |
| Thiamin | mg | 0.64 | 0.09 | 0.06 |
| Riboflavin | mg | 0.85 | 0.12 | 0.09 |
| Niacin | mg | 8 | 1.1 | 0.8 |
| Niacin equivalent | mg NE | 14.8 | 2.1 | 1.5 |
| Vitamin B₆ | mg | 0.57 | 0.08 | 0.06 |
| Folic Acid | µg | 215 | 30.4 | 21.5 |
| Vitamin B₁₂ | µg | 1.3 | 0.18 | 0.13 |
| Biotin | µg | 16.2 | 2.3 | 1.6 |
| Pantothenic Acid | mg | 5.8 | 0.82 | 0.58 |
| Choline | mg | 170 | 24 | 17 |
| Inositol | mg | 170 | 24 | 17 |

| ***Minerals*** | | ***Per 100g Powder*** | ***Per 100 kcal **** | ***Per 100ml***** |
|---|---|---|---|---|
| Sodium | mg | 318 | 45 | 31.8 |
| | mmol | 13.8 | 2 | 1.4 |
| Potassium | mg | 925 | 131 | 92.5 |
| | mmol | 23.7 | 3.4 | 2.4 |
| Chloride | mg | 547 | 77.4 | 54.7 |
| | mmol | 15.6 | 2.2 | 1.6 |
| Calcium | mg | 649 | 91.8 | 64.9 |
| Phosphorus | mg | 500 | 70.7 | 50 |
| Magnesium | mg | 81.7 | 11.6 | 8.2 |

| ***Trace Elements*** | | ***Per 100g Powder*** | ***Per 100 kcal* *** | ***Per 100ml***** |
|---|---|---|---|---|
| Iron | mg | 10.7 | 1.5 | 1.1 |
| Copper | µg | 710 | 100 | 71 |
| Zinc | mg | 5.7 | 0.81 | 0.57 |
| Manganese | mg | 0.92 | 0.13 | 0.09 |
| Iodine | µg | 127 | 18 | 12.7 |
| Molybdenum | µg | 31.8 | 4.5 | 3.2 |
| Selenium | µg | 19 | 2.7 | 1.9 |
| Chromium | µg | 31.8 | 4.5 | 3.2 |
| Fluoride | mg | 0.85 | 0.12 | 0.09 |

This composition is prepared by mixing a blend of palm oils and soybean oils with an aqueous phase comprising the intact milk proteins, maltodextrins, vitamins and minerals and other components like flavors, homogenizing the mixture and spray-drying. Optionally additional heating and cooling steps as well as additional homogenization methods can be included. The product comprises less than 5wt% trans fatty acids and demonstrates relatively superior resistance against fat oxidation, in terms of formation of oxidation components and off flavors.

### Example 2: New formula for paediatric infants

Product as in example 1, however modified for specific components:

| *Component* | *per 100 g powder* |
|---|---|
| Vitamin B6 | 0.9 mg |
| Carnitine | 50 mg (as acetyl-L-carnitine) |
| Taurine | 60 mg |
| Pantothenic acid | 4 mg |

## Claims

1. A solid nutritional product ***which is a powder,*** comprising proteins, lipids and digestible carbohydrates, these components originating from at least two different sources, which provides 2520-3080 kiloJoule per 100 g dry mass, in which the weight ratio of lipids to the sum of proteins and digestible carbohydrates is ***2.7-3.4 to 1, and in which the lipids comprise 16-40 wt. % of polyunsaturated fatty acids, on fatty acid basis.***

2. The nutritional product according to ***claim 1,*** in which the lipids comprise 23-50 wt.% of palmitic acid, on fatty acid basis.

3. The nutritional product according to any one of the preceding claims, in which the lipids comprise 10-25 wt.% of one or more of phospholipids, lyso-phospholipids, diacylglycerides and monoglycerides.

4. The nutritional product according to ***any one of the preceding claims,*** in which the polyunsaturated fatty acids comprise 1-10 wt% of docosahexaenoic acid.

5. The nutritional product according to any one of the preceding claims, in which the proteins comprise at least 20 wt.% of α-lactalbumin.

6. The nutritional product according to any one of the preceding claims, in which the carbohydrates comprise 20-90 wt.% of a source of galactose and/or ribose units.

7. The nutritional product according to any one of the preceding claims, which contains 6-100 mg of nucleotides per 100 g of dry matter.

8. The nutritional product according to any one of the preceding claims, which contains more than 25 mg of carnitine per 100 g of dry matter.

9. The nutritional product according to any one of the preceding claims, which contains 40 mg - 1.8 g of non-digestible oligosaccharides having 3-20 monosaccharide units, comprising at least two different monosaccharide units selected from galactose, fructose, fucose, mannose, arabinose, glucuronic acid, galacturonic acid, sialic acid, and N-acetyl glucosamine, per 100 g of dry matter.

10. The nutritional product according to any one of claims ***1-9*, characterized in that** at least 50% of the dry mass is soluble when dissolved as 10% in water at 20 °C.

11. A liquid nutritional product obtained by reconstituting the product of any one of claims ***1-9*** with water.

12. The liquid product of claim ***11*,** comprising added vitamins and/or added trace elements and having an energy density of 1.2-1.8 kcal/ml.

## Patentansprüche

1. Festes oder halbfestes Ernährungsprodukt, welches ein Pulver ist, umfassend Proteine, Lipide und verdauliche Kohlenhydrate, wobei diese Komponenten aus mindestens zwei verschiedenen Quellen stammen, welches 2520-3080 kJ pro 100 g Trockenmasse verschafft, wobei das Gewichtsverhältnis von Lipiden zu der Summe aus Proteinen und verdaulichen Kohlenhydraten 2,7-3,4 zu 1 beträgt, und wobei die Lipide 16-40 Gew.-% mehrfach ungesättigte Fettsäuren, auf Fettsäurebasis, umfassen.

2. Ernährungsprodukt nach Anspruch 1, wobei die Lipide 23-50 Gew.-% Palmitinsäure, auf Fettsäurebasis, umfassen.

3. Ernährungsprodukt nach einem der vorangehenden Ansprüche, wobei die Lipide 10-25 Gew.-% eines oder mehrerer aus Phospholipiden, Lyso-Phospholipiden, Diacylglyceriden und Monoglyceriden umfassen.

4. Ernährungsprodukt nach einem der vorangehenden Ansprüche, wobei die mehrfach ungesättigten Fettsäuren 1-10 Gew.-% Docosahexaensäure umfassen.

5. Ernährungsprodukt nach einem der vorangehenden Ansprüche, wobei die Proteine mindestens 20 Gew.-% α-Lactalbumin umfassen.

6. Ernährungsprodukt nach einem der vorangehenden Ansprüche, wobei die Kohlenhydrate 20-90 Gew.-% einer Quelle an Galactose- und/oder Ribose-Einheiten umfassen.

7. Ernährungsprodukt nach einem der vorangehenden Ansprüche, welches 6-100 mg Nucleotide pro 100 g Trockenmasse enthält.

8. Ernährungsprodukt nach einem der vorangehenden Ansprüche, welches mehr als 25 mg L-Carnitin pro 100 g Trockenmasse enthält.

9. Ernährungsprodukt nach einem der vorangehenden Ansprüche, das 40 mg - 1,8 g nichtverdauliche Oligosaccharide mit 3-20 Monosaccharideinheiten enthält, umfassend mindestens zwei verschiedene Monosaccharideinheiten, gewählt aus Galactose, Fructose, Fucose, Mannose, Arabinose, Glucuronsäure, Galacturonsäure, Sialinsäure und N-Acetylglucosamin, pro 100 g Trockenmasse.

10. Ernährungsprodukt nach einem der Ansprüchen 1-9, **dadurch gekennzeichnet, dass** mindestens 50% der Trockenmasse löslich sind, wenn als 10%ig in Wasser bei 20°C gelöst.

11. Flüssiges Ernährungsprodukt, erhalten durch Rekonstituieren des Produkts nach mindestens einem der Ansprüche 1-9 mit Wasser.

12. Flüssiges Produkt nach Anspruch 11, das zugesetzte Vitamine und/oder zugesetzte Spurenelemente umfasst, und das eine Energiedichte von 1,2-1,8 kcal/ml aufweist.

## Revendications

1. Produit nutritionnel solide ou semi-solide, qui est un poudre, comportant des protéines, des lipides et des hydrates de carbone digestibles, ces composants provenant d'au moins deux sources différentes, le produit apportant de 2520 à 3080 kilojoules pour 100 g de masse sèche, dans lequel le rapport en poids de lipides par rapport à la somme des protéines et des hydrates de carbone digestibles est compris entre 2,7 et 3,4 à 1, et dans lequel les lipides comprennent de 16 à 40 % en poids d'acides gras polyinsaturés, en prenant comme base les acides gras.

2. Produit nutritionnel selon la revendication 1, dans lequel les lipides comprennent de 23 à 50 % en poids d'acide palmitique, en prenant comme base les acides gras.

3. Produit nutritionnel selon l'une quelconque des revendications précédentes, dans lequel les lipides comprennent de 10 à 25 % en poids d'un ou plusieurs de phospholipides, lysophospholipides, diacylglycérides et monoglycérides.

4. Produit nutritionnel selon l'une quelconque des revendications précédentes, dans lequel les acides gras polyinsaturés comprennent de 1 à 10 % en poids d'acide docosahexaénoïque.

5. Produit nutritionnel selon l'une quelconque des revendications précédentes, dans lequel les protéines comprennent au moins 20 % en poids de α-lactalbumine.

6. Produit nutritionnel selon l'une quelconque des revendications précédentes, dans lequel les hydrates de carbone comprennent de 20 à 90 % en poids d'une source d'unités de galactose et/ou de ribose.

7. Produit nutritionnel selon l'une quelconque des revendications précédentes, qui comprend de 6 à 100 mg de nucléotides pour 100 g de matière sèche.

8. Produit nutritionnel selon l'une quelconque des revendications précédentes, qui comprend plus que 25 mg de L-carnitine pour 100 g de matière sèche.

9. Produit nutritionnel selon l'une quelconque des revendications précédentes, qui comprend de 40 mg à 1,8 g d'oligosaccharides non digestibles présentant 3 à 20 unités de monosaccharide, comprenant au moins deux unités de monosaccharide différentes choisies parmi le galactose, le fructose, le fucose, le mannose, l'arabinose, l'acide glucuronique, l'acide galacturonique, l'acide sialique, et la N-acétyl glucosamine, pour 100 g de matière sèche.

10. Produit nutritionnel selon l'une quelconque des revendications 1 à 9, **caractérisé en ce qu'**au moins 50 % de la masse sèche est soluble lorsque dissoute à 10 % dans de l'eau à 20°C.

11. Produit nutritionnel liquide obtenu par reconstitution du produit selon l'une quelconque des revendications 1 à 9 avec de l'eau.

12. Produit liquide selon la revendication 11, comportant des vitamines ajoutées et/ou des oligoéléments supplémentaires et présentant une densité d'énergie comprise entre 1,2 et 1,8 kcal/ml.
